# EUROPÄISCHE PATENTSCHRIFT

(11) **EP 1 646 322 B1**
(45) Veröffentlichungstag und Bekanntmachung des Hinweises auf die Patenterteilung: **10.12.2008**
(21) Anmeldenummer: 04763190.8
(22) Anmeldetag: 13.07.2004
(51) Int. Cl.: A61B 17/52

(54) **MEDIZINTECHNISCHE EINRICHTUNG**
MEDICAL DEVICE
DISPOSITIF MEDICO-TECHNIQUE

(30) Priorität: 16.07.2003 DE 10332475
(43) Veröffentlichungstag der Anmeldung: 19.04.2006
(73) Patentinhaber: Zimmermann, Joachim, 88142 Wasserburg a.B. (DE)
(72) Erfinder: Zimmermann, Joachim, 88142 Wasserburg a.B. (DE)
(74) Vertreter: Weiss, Peter
(86) Internationale Anmeldenummer: PCT/EP2004/007723
(87) Internationale Veröffentlichungsnummer: WO 2005/006996

(56) Entgegenhaltungen:
- WO-A-01/07111
- DE-A- 3 139 811
- US-A- 3 805 777
- US-A- 4 102 998
- US-B1- 6 398 713

## Beschreibung

Die Erfindung betrifft eine medizintechnische Einrichtung zur Behandlung von Lebewesen mit einem Magnetfeld, wobei die Einrichtung in eine Vagina einer Frau oder in einen Analbereich eines Lebewesens eingesetzt werden kann, umfassend ein flexibles Element bestehend aus einem inerten Schlauch, welcher zu einem Ring geformt ist und in welchen magnetisierte Elemente eingeführt sind.

### STAND DER TECHNIK

In den letzten Jahren haben Therapiesysteme mit magnetischen Feldern, insbesondere mit pulsierenden magnetischen Feldern (PEMF) wissenschaftliche Anerkennung und bei den Patienten sehr grossen Anklang gefunden. Entsprechende Geräte und Einrichtungen finden vor allem Anwendung bei der Therapie von Schmerzzuständen, die auf eine übermässige Degeneration (= Abnutzung), zum Beispiel Gelenkschmerzen, zurückzuführen sind. Ferner werden sie eingesetzt bei gestörter Durchblutung, zum Beispiel bei Morbus Sudeck, Raynaud-Krankheit, Durchblutungsstörung durch Zuckerkrankheit.

Entsprechende Geräte werde in der Regel mit einer Grossfeldspule angeboten, in welcher ein Patient mit seinem ganzen Körper Platz hat. Ferner gibt es auch Impulsgeneratoren mit Flachapplikatoren, welche ein magnetisches Streufeld erzeugen, das den Körper ganz durchdringt. Es ist in Bezug auf die Frequenz und Intensitäten identisch mit dem pulsierenden Magnetfeld der Grossfeldspule. Allerdings ist das konzentrische Magnetfeld der Grossfeldspule umfassender.

### AUFGABE

Der vorliegenden Erfindung liegt die Aufgabe zugrunde, die Anwendung der Magnetfeldtherapie zu erweitern.

### LÖSUNG DER AUFGABE

Zur Lösung dieser Aufgabe führt, dass die Elemente Kugeln und in sich anziehender Ausrichtung eingefüllt sind, oder dass die Elemente Ringe und in gegensätzlicher Ausrichtung aneinandergereiht sind.

Das bedeutet, dass die Magnetfeldtherapie nicht nur von aussen auf den Körper des Lebewesens Einfluss ausübt, sondern direkt in das Innere einstrahlt, ohne die Aussenhaut durchqueren zu müssen. In der Praxis hat sich herausgestellt, dass hierdurch eine wesentlich direktere Einflussnahme auf die inneren Organe genommen werden kann.

## Patentansprüche

1. Medizintechnische Einrichtung zur Behandlung von Lebewesen mit einem Magnetfeld, wobei die Einrichtung in eine Vagina einer Frau oder in einen Analbereich eines Lebewesens eingesetzt werden kann, umfassend ein flexibles Element bestehend aus einem inerten Schlauch, welcher zu einem Ring geformt ist und in welchen magnetisierte Elemente eingeführt sind,
**dadurch gekennzeichnet,**
**dass** die Elemente Kugeln und in sich anziehender Ausrichtung eingefüllt sind, oder dass die Elemente Ringe und in gegensätzlicher Ausrichtung aneinandergereiht sind.

## Claims

1. Medical device for the treatment of living creatures by means of a magnetic field, wherein the device may be inserted into the vagina of a female or into an anal region of a living creature, comprising a flexible element made from an inert tube, which is formed into a ring and into which magnetized elements are introduced,
**characterized in**
**that** the elements are spheres and are introduced in mutually attractive alignment, or that the elements are rings and are butt-mounted in opposing alignment.

## Revendications

1. Dispositif médico-technique pour le traitement d'êtres vivants par un champ magnétique, le dispositif pouvant être placé dans le vagin d'une femme ou dans une zone anale d'un être vivant, comprenant un élément flexible composé d'un tube inerte qui peut être formé pour obtenir un anneau et dans lequel sont introduits des éléments magnétiques,
**caractérisé par le fait**
**que** les éléments sont des billes et sont introduits selon une orientation dans laquelle ils s'attirent, ou que les éléments sont des anneaux et sont juxtaposés selon une orientation opposée.
